Europäisches Patentamt

European Patent Office

Office européen des brevets

(19)

(11) Numéro de publication: **0 201 412**
**B1**

(12) # FASCICULE DE BREVET EUROPEEN

(45) Date de publication du fascicule du brevet:
**26.07.89**

(21) Numéro de dépôt: **86400937.8**

(22) Date de dépôt: **29.04.86**

(51) Int. Cl.⁴: **A 23 G 3/00,** A 23 G 3/26,
A 61 K 9/36

(54) **Produit de confiserie ou pharmaceutique à revêtement sans sucre obtenu par dragéification dure et son procédé de préparation.**

(30) Priorité: **30.04.85 FR 8506579**

(43) Date de publication de la demande:
**12.11.86 Bulletin 86/46**

(45) Mention de la délivrance du brevet:
**26.07.89 Bulletin 89/30**

(84) Etats contractants désignés:
**BE CH DE GB IT LI NL SE**

(56) Documents cités:
**FR-A- 2 467 597**
**FR-A- 2 486 364**
**FR-A- 2 522 250**
**US-A- 4 238 510**
**US-A- 4 293 570**
**US-A- 4 357 314**
**US-A- 4 381 318**

(73) Titulaire: **Roquette Frères, F-62136 Lestrem (FR)**

(72) Inventeur: **Boursier, Bernard, 63, route d'Estaires,
F-62138 Violaines (FR)**

(74) Mandataire: **Koch, Gustave et al, Cabinet
PLASSERAUD 84, rue d'Amsterdam, F-75009 Paris (FR)**

ACTORUM AG

## Description

L'invention a pour objet un produit de confiserie ou pharmaceutique à revêtement sans sucre obtenu par dragéification dure.

Elle vise également le procédé de préparation de ce produit.

La dragéification dite «dure» se distingue de la dragéification dite «tendre» en ce sens que la matière constitutive du revêtement est cristallisée, l'eau contenue dans ladite matière étant évaporée.

On connaît déjà des produits de confiserie, c'est-à-dire des chewing-gums, pâtes à mâcher, bonbons et réglisses, et des produits pharmaceutiques sous forme de tablettes, comprimés ou bonbons à revêtement sans sucre obtenus par dragéification dure.

Dans ces produits, le revêtement sans sucre obtenu par dragéification dure est à base de xylitol et, dans une moindre mesure, à base de mannitol.

Plus récemment, la Société Demanderesse a mis au point un procédé de dragéification dure objet du brevet français No 2.467.597 et permettant, contre toute attente, de réaliser un revêtement du genre en question à base de sorbitol.

Ce procédé et le produit résultant donnent toute satisfaction mais, en raison des besoins sans cesse croissants des industries pharmaceutiques et de la confiserie en produits à revêtement sans sucre obtenus par dragéification dure, la Société Demanderesse a poursuivi ses recherches et a eu le mérite de trouver qu'il était possible de réaliser un revêtement du genre en question à base de maltitol, alors que les revêtements de nature essentiellement cristalline et à base de maltitol connus auparavant étaient obtenus par dragéification tendre, ainsi qu'il résulte du brevet français No 2.486.364.

Il s'ensuit que le produit de confiserie ou pharmaceutique à revêtement sans sucre de nature essentiellement cristalline, conforme à l'invention, est caractérisé par le fait que ledit revêtement est obtenu par dragéification dure et qu'il comporte au moins 90 % en poids de maltitol.

Et le procédé de préparation du susdit produit est caractérisé par le fait que l'on applique un sirop de maltitol

– d'une richesse en maltitol supérieure à 92 %, de préférence à 95 % en poids sur matière sèche, et plus préférentiellement supérieure à 96 %,

– d'une teneur en matières sèches de 50 à 70 % en poids, de préférence de 55 à 65 % en poids et

– d'une température inférieure à 70 °C et, de préférence de 45 à 65 °C, sur un lit en mouvement de noyaux à dragéifier, la température régnant dans ledit lit de noyaux en mouvement étant inférieure à 55 °C et de préférence de 20 à 40 °C, l'ensemble des susdites conditions étant choisies, à l'intérieur des gammes susmentionnées, de telle manière que, lorsque le sirop de maltitol arrive au contact des noyaux à dragéifier, c'est-à-dire à la température régnant au sein du lit de noyaux en mouvement, il se trouve à un niveau de saturation de 0,70 à 0,90 et, de préférence, de 0,75 à 0,90.

Le fait que l'ensemble des susdites conditions, sélectionnées comme indiqué, conduise à un revêtement de dragéification dure à base de maltitol de qualité satisfaisante était d'autant plus inattendu que, contrairement à ce qui se passe dans le cas de la dragéification dure au saccharose – disaccharide comme le maltitol et de comportement voisin à celui du maltitol dans de nombreux autres domaines – la sursaturation entraînée par ces conditions n'est pas élevée alors que l'on devait normalement s'attendre à ce que la sursaturation nécessaire à l'obtention d'une dragéification dure de bonne qualité soit voisine dans le cas des deux disaccharides en question.

On rappelle que, par l'expression «niveau de saturation», on désigne le rapport, pour une température donnée, de la concentration du sirop exprimée en grammes de maltitol pour $100 \text{ cm}^3$ d'eau, sur la limite de solubilité du maltitol, à la température donnée, également exprimée en grammes de maltitol pour $100 \text{ cm}^3$ d'eau.

Dans le tableau I, on a réuni pour un certain nombre de températures, les valeurs de la limite de solubilité du maltitol (exprimée en g/100 cm³).

Tableau I

| t °C | Limite de solubilité (en g/100 cm³) |
|---|---|
| 20 | 153 |
| 25 | 170 |
| 30 | 181 |
| 35 | 193 |
| 40 | 210 |
| 45 | 226 |
| 50 | 241 |

L'invention pourra être encore mieux comprise à l'aide du complément de description qui suit et des exemples qui sont donnés en rapport avec des modes de réalisation avantageux.

Se proposant, par conséquent, de fabriquer les produits de dragéification conformes à l'invention, on s'y prend comme suit ou de façon équivalente.

Des noyaux à dragéifier du type confiserie ou produit pharmaceutique, «sans sucre» ou non, sont maintenus sous forme d'un lit en mouvement dans une cuve tournante de dragéification de type conventionnel et équipée de moyens de contrôle de la température intérieure.

On applique, par exemple par pulvérisation, sur ladite masse de noyaux en mouvement un sirop de maltitol dont la température est inférieure à 70 °C et, de préférence, de 45 à 65 °C.

Le sirop de maltitol mis in œuvre présente une concentration en matières sèches de 50 à 70 % en poids, de préférence de 55 à 65 % en poids.

La richesse de ce sirop en maltitol est supérieure à 92 %, de préférence à 95 %, et, plus préférentiellement encore, à 96% en poids sur matière sèche.

La température régnant dans le lit de noyaux en mouvement est maintenue à une valeur inférieure à 55°C, de préférence de 20 à 40°C.

On choisit la concentration en matières sèches du sirop de maltitol et la température du lit de noyaux à l'intérieur des susdites gammes de telle manière que, lorsque le sirop de maltitol arrive au contact des noyaux à enrober, il se trouve à un niveau de saturation de 0,70 à 0,90, de préférence de 0,75 à 0,90.

Un exemple de sirop de maltitol ayant donné satisfaction est celui de composition suivante:
maltitol: 97,1% en poids
sorbitol: 1,1% en poids
maltotriitol: 1,8% en poids.

L'enrobage est effectué par cycles successifs comprenant chacun une première phase d'addition du sirop de maltitol sur le lit de noyaux et une seconde phase pendant laquelle on arrête l'addition tout en maintenant la rotation de la cuve et la température régnant au sein de la masse de noyaux, l'enveloppe dont ont été enrobés les noyaux étant séchée et polie au cours de cette phase.

L'épaisseur de l'enveloppe est choisie en fonction notamment du noyau à dragéifier ou des effets recherchés.

Pour fixer les idées, on peut réaliser un revêtement de par exemple environ 0,5 mm d'épaisseur en effectuant successivement de 15 à 30 additions, ce qui, compte tenu de la durée d'une addition et du laps de temps séparant la fin d'une addition du début de la suivante conduit à une durée totale de l'opération de dragéification de l'ordre de 3 à 7 heures.

Les moyens mis en œuvre pour maintenir la température au sein de la masse de noyaux en mouvement à la valeur souhaitée, peuvent être constitués par un dispositif d'insufflation d'air chaud de température contrôlée.

Grâce aux conditions d'enrobage conformes à l'invention, on peut obtenir une surface lisse et régulière.

Les produits dragéifiés ainsi obtenus présentent par rapport aux produits dragéifiés par dragéification dure à revêtement sans sucre de l'art antérieur, notamment ceux à base de sorbitol, l'avantage
– d'être d'une blancheur très agréable à la vue,
– d'être d'un goût plus sucré et
– d'être plus croustillants.

Pour optimiser les résultats, on préfère appliquer aux noyaux traités et avant la dragéification proprement dite, une couche de gommage qui peut être, par exemple, à base de gomme arabique, de gélatine ou produit équivalent.

Il est possible d'ajouter au sirop de maltitol mis en œuvre, divers additifs comme des colorants, des arômes ou des agents améliorant l'état de surface tels que la cire d'abeille ou de la cire de carnauba.

Parmi les additifs, on peut citer le dioxyde de titane, parmi les arômes, ceux de menthe, orange et citron.

Dans le choix des produits pharmaceutiques et de confiserie constitutifs du noyau à dragéifier, on fera appel volontiers, en raison du fait que le revêtement appliqué est «sans sucre», aux produits «sans sucre», notamment à base de sorbitol, de xylitol, mannitol, maltitol et de sirops de glucose hydrogénés de marque LYCASIN®.

Lorsque le noyau à dragéifier contient un sucre fermentescible, le revêtement obtenu conformément à l'invention atténue le caractère cariogène de l'ensemble et lui confère de toute façon les qualités inhérentes au maltitol.

Les produits de dragéification conformes à l'invention peuvent présenter une surface lisse essentiellement exempte d'imperfections et cristalline sur toute l'épaisseur de l'enveloppe; ils sont stables, même dans des atmosphères à humidité élevée et ils présentent en outre une saveur sucrée très agréable.

Dans les exemples qui suivent, on envisage successivement:
– la préparation d'un chewing-gum dragéifié conforme à l'invention et, à cet égard, la détermination des conditions optimales de la dragéification dure à l'aide du maltitol,
– la préparation d'un produit dragéifié de confiserie conforme à l'invention du type «produit de compression»,
– la préparation d'un bonbon sucre cuit du type «sans sucre» dragéifié conforme à l'invention.

Exemple 1
Dragéification dure au maltitol d'un chewing-gum du type «sans sucre».
La composition des plaquettes de chewing-gum utilisées pour cet exemple est la suivante:
– 28 parties en poids d'une gomme base constituée par celle que commercialise la Société DREYFUS sous l'appellation «EXTRA PALOJA»,
– 20 parties en poids d'un sirop de glucose hydrogéné (concentré à 85% de matières sèches ou MS) constitué par celui que commercialise la Société ROQUETTE FRERES sous la marque LYCASIN® 80/55,
– 50,8 parties en poids de sorbitol poudre constitué par celui que commercialise la Société ROQUETTE FRERES sous la marque NEOSORB P60®,
– 1,2 partie en poids d'arômes et de colorants.

Ces plaquettes sont préparées en suivant la séquence opérationnelle indiquée ci-après:

– après réchauffage à 75°C, la gomme base est malaxée dans un pétrin du type KUSTNER muni d'une circulation d'eau chaude, en présence de la phase liquide constituée par le sirop de glucose hydrogéné; au cours de cette étape, la phase solide constituée par le sorbitol poudre est ajoutée progressivement par petites quantités; on incorpore finalement les arômes et colorants,

– après poudrage de la pâte au mannitol, on effectue le laminage de la pâte et son découpage en coussinets de forme rectangulaire et d'un poids nominal moyen de 0,9 g.

On place 500 g de ces coussinets dans une drageuse de laboratoire du type «LILLIPUT» commercialisée par la Société FROGERAIS, équipée d'une soufflerie d'air régulé permettant de maintenir constante la température du lit de coussinets, et d'une sonde thermométrique positionnée dans le lit.

La drageuse est animée d'une vitesse de rotation comprise entre 22 et 25 tours/minute.

Le sirop d'enrobage à base de maltitol est maintenu à une température constante dans un bain-marie thermorégulé et il est ajouté par charges successives (cycles) de 13 à 30 g de sirop, l'introduction se faisant en quelques secondes toutes les 3 à 7 minutes. Ce temps, séparant la fin d'une introduction de l'introduction suivante, permet de réaliser la cristallisation du maltitol et l'évaporation de l'eau.

Le sirop de maltitol utilisé pour l'enrobage est essentiellement constitué de maltitol et d'une certaine teneur en sorbitol et maltotriitol.

Avant de déterminer par quatre séries d'expériences successives les conditions optimales du procédé de dragéification dure au maltitol conforme à l'invention, on a procédé à deux expériences comparatives, consistant à opérer dans les conditions de la dragéification dure au saccharose et au sorbitol.

a) Expériences comparatives

Le sirop de maltitol utilisé est d'une richesse de 95% en maltitol, de 1,6% de sorbitol et de 3,4% en maltotriitol.

Les conditions de la première expérience comparative (celles de la dragéification dure au saccharose) et de la seconde (celles de la dragéification dure au sorbitol), ainsi que les résultats obtenus, sont précisés dans le tableau II.

Tableau II

| N° de l'expérience | 1 | 2 |
|---|---|---|
| richesse du sirop | 95% | 95% |
| matière sèche du sirop | 80% | 70% |
| température du lit en mouvement | 30 °C | 30 °C |
| température du sirop | 80 °C | 40 °C |
| nombre de cycles | 10–15 | 10–15 |
| poids de sirop mis en œuvre en grammes environ | 180 | 180 |

Dans les deux cas, les produits dragéifiés obtenus ne donnent pas satisfaction; des défauts de surface très prononcés sont apparus et s'expliquent par une cristallisation très lente et irrégulière, ce qui montre qu'il n'est pas possible d'obtenir une dragéification dure au maltitol répondant aux exigences de la pratique en reprenant simplement, ce qui était a priori logique, les conditions connues avant tout pour la dragéification dure au saccharose.

b) Expériences effectuées en vue de la détermination des conditions optimales de richesse en maltitol du sirop utilisé.

A l'aide de quatre sirops de maltitol de richesses différentes, on a réalisé, dans des conditions par ailleurs identiques et à l'aide de l'appareillage précédemment décrit, les expériences 3 à 6 dont les conditions et les résultats sont reportés dans le tableau III.

La ligne réservée aux appréciations permet, à l'aide du symbolisme suivant, de définir la qualité de la dragéification obtenue.

| | |
|---|---|
| mauvais | ooo |
| très médiocre | oo |
| médiocre | o |
| acceptable | x |
| bon | xx |
| très bon | xxx |

Les quatre sirops de maltitol mis en œuvre sont à 60% de matières sèches et présentent une richesse en maltitol respectivement de 92, 95, 96 et 97%.

Tableau III

| N° de l'expérience | 3 | 4 | 5 | 6 |
|---|---|---|---|---|
| richesse du sirop | 92% | 95% | 96% | 97% |
| matière sèche du sirop | 60% | 60% | 60% | 60% |
| température du lit en mouvement | 30 °C | 30 °C | 30 °C | 30 °C |
| température du sirop | 50 °C | 50 °C | 50 °C | 50 °C |
| nombre de cycles | 10–15 | 10–15 | 10–15 | 10–15 |
| poids de sirop mis en œuvre | 250 g | 250 g | 250 g | 250 g |
| niveau de saturation | 0,76 | 0,79 | 0,80 | 0,83 |
| appréciation | oo | o | xx | xxx |

On constate qu'à 96 et 97% de richesse, les dragéifications obtenues sont tout à fait correctes, la cristallisation du maltitol s'est réalisée de manière très régulière et nécessite seulement un temps de séchage légèrement supérieur dans le cas du sirop à 96% de richesse. Les produits correspondants présentent une croustillance et une saveur sucrée agréable.

Pour les sirops à 95 et 92% de richesse, dans ces conditions, les chewing-gums ont tendance à coller entre eux, la cristallisation est difficile et les temps de séchage sont longs, ces défauts s'accentuant dans le cas du sirop à 92%.

c) Expériences effectuées en vue de la détermination des conditions optimales de teneur en matières sèches du sirop utilisé.

Tableau IV

| N° de l'expérience | 7 | 8 | 9 | 10 | 11 | 12 |
|---|---|---|---|---|---|---|
| richesse du sirop | 97% | 97% | 97% | 97% | 97% | 97% |
| matière sèche du sirop | 45% | 50% | 55% | 60% | 65% | 70% |
| nombre de cycles | 10−15 | 10−15 | 10−15 | 10−15 | 10−15 | 10−15 |
| poids de sirop mis en œuvre | 333 g | 300 g | 275 g | 250 g | 230 g | 214 g |
| niveau de saturation | 0,44 | 0,53 | 0,80 | 0,83 | 0,99 | 1,25 |
| appréciation | o | x | xx | xxx | x | oo |

On obtient de bons résultats pour des teneurs en matières sèches de 50 à 65% et de très bons résultats pour des matières sèches de 55 à 60%.

Au-delà de 65%, la cristallisation est irrégulière et des défauts de surface apparaissent; au-dessous de 50%, les chewing-gums sont collants et les temps de séchage importants.

d) Expériences effectuées en vue de la détermination des conditions optimales du point de vue de la température régnant au sein du lit de noyaux en mouvement.

Un sirop d'une richesse de 97% en maltitol a été testé pour six teneurs en matières sèches différentes, à savoir respectivement 45, 50, 55, 60, 65 et 70%, ce qui correspond aux expériences 7 à 12.

Les conditions et les résultats de ces essais sont donnés dans le tableau IV.

On met en œuvre un sirop de maltitol à 60% de matières sèches et d'une richesse de 97% en maltitol.

Dans les 6 expériences (n° 13 à 18) effectuées avec ce sirop, la dragéification a été réalisée à autant de températures différentes au sein du lit en mouvement, le sirop d'alimentation étant maintenu à 50 °C.

Les conditions et les résultats de ces essais sont reportés dans le tableau V.

Tableau V

| N° de l'expérience | 13 | 14 | 15 | 16 | 17 | 18 |
|---|---|---|---|---|---|---|
| température du lit | 10 °C | 15 °C | 20 °C | 30 °C | 40 °C | 50 °C |
| nombre de cycles | 10−15 | 10−15 | 10−15 | 10−15 | 10−15 | 10−15 |
| poids de sirop mis en œuvre | 250 g | 250 g | 250 g | 250 g | 250 g | 250 g |
| niveau de saturation | 1,07 | 1 | 0,95 | 0,81 | 0,69 | 0,60 |
| appréciation | oo | o | x | xxx | xx | o |

Il apparaît à l'examen de ces résultats que l'on a intérêt à maintenir la température du lit entre 20 et 40 °C.

Dans ces conditions, les produits obtenus présentent une bonne cristallinité de la couche ainsi qu'une excellente stabilité au stockage.

En raison du ramollissement des coussinets, on n'a pu procéder à aucune expérience à des températures supérieures à 50 °C.

e) Expériences effectuées en vue de la détermination des conditions optimales du point de vue de la température du sirop.

On a effectué six expériences (19 à 24) en utilisant le sirop des expériences 13 à 18, la température du lit ayant été maintenue à 30 °C.

Les autres paramètres des essais et les résultats obtenus sont regroupés dans le tableau VI.

Tableau VI

| N° de l'expérience | 19 | 20 | 21 | 22 | 23 | 24 |
|---|---|---|---|---|---|---|
| température du sirop | 40 °C | 45 °C | 50 °C | 60 °C | 65 °C | 70 °C |
| nombre de cycles | 10−15 | 10−15 | 10−15 | 10−15 | 10−15 | 10−15 |
| poids de sirop mis en œuvre | 250 g | 250 g | 250 g | 250 g | 250 g | 250 g |
| niveau de saturation | 0,81 | 0,81 | 0,81 | 0,81 | 0,81 | 0,81 |
| appréciation | o | xx | xxx | xx | o | oo |

Au vu de ces résultats, on constate qu'il est préférable de travailler dans les conditions de l'expérience avec un sirop de maltitol maintenu à une température de 45 à 60 °C.

Au-delà de 60 °C, après ajout du sirop, dans un premier temps, les chewing-gums collent entre eux puis il se produit une cristallisation rapide mais irrégulière; au-dessous de 40 °C, la répartition de la couche cristalline devient moins bonne et les temps de séchage augmentent sensiblement.

A l'examen de l'ensemble des résultats obtenus dans les expériences 3 à 24, il apparaît que, pour obtenir une dragéification de qualité, il y a lieu d'opérer à un niveau de saturation de 0,7 à 0,9.

Exemple 2

Dragéification dure de produits de compression.

Les noyaux à dragéifier ont été préparés à l'aide d'une presse rotative à haut rendement du type P 1000, commercialisée par la Société WILHELM FETTE GmbH.

Le produit utilisé est constitué par du sorbitol poudre de marque NEOSORB® 20/60, aromatisé à la menthe et comportant 0,3% en poids d'un lubrifiant constitué par du stéarate de magnésium.

Les comprimés de sorbitol ainsi préparés, d'un poids moyen de 0,5 g, sont ronds et biconcaves.

On en utilise 500 g.

La dragéification est conduite au moyen de l'appareillage précédemment utilisé pour la dragéification des chewing-gums; on a chaque fois procédé à 10-15 ajouts, espacés de 10 minutes chaque fois, de 15 g de maltitol.

Comme le montrent les résultats des 10 expériences (25 à 34) réalisées avec ces comprimés et rassemblés dans le tableau VII, la dragéification de ces comprimés donne des produits de qualité, dans la mesure où l'on maintient les conditions de niveau de saturation établies pour la dragéification des chewing-gums.

Les produits obtenus présentent une bonne cristallinité de couche, une bonne stabilité au stockage et une certaine croustillance lors de la dégustation.

Tableau VII

| N° de l'expérience | 25 | 26 | 27 | 28 | 29 | 30 | 31 | 32 | 33 | 34 |
|---|---|---|---|---|---|---|---|---|---|---|
| richesse du sirop | 97% | 97% | 97% | 96% | 97% | 97% | 97% | 92% | 97% | 97% |
| matière sèche du sirop | 45% | 60% | 55% | 60% | 60% | 50% | 45% | 60% | 60% | 60% |
| température du lit | 30 °C | 30 °C | 30 °C | 30 °C | 40 °C | 30 °C | 30 °C | 30 °C | 50 °C | 60 °C |
| niveau de saturation | 0,44 | 0,83 | 0,8 | 0,8 | 0,69 | 0,53 | 0,44 | 0,76 | 0,60 | 0,48 |
| appréciation | o | xxx | xxx | xx | xx | x | o | oo | o | ooo |

Exemple 3

Dragéification dure de bonbons «sucre cuit» du type «sans sucre».

On a préparé des bonbons de sucre cuit en évaporant jusqu'à une humidité résiduelle de 0,5% un hydrolysat d'amidon hydrogéné, commercialisé sous la marque LYCASIN® 80/55.

Ces bonbons ont été mis sous forme de sphères d'un diamètre moyen de 1,5 cm et d'un poids moyen de 0,9 g.

Pour la dragéification, on a utilisé le matériel et les conditions de mise en œuvre décrites plus haut. La température du lit a été dans tous les cas maintenue à une valeur inférieure ou au plus égale à 60 °C, cette valeur étant imposée par la matière constitutive des noyaux.

Le sirop d'une richesse de 97% en maltitol est ajouté à 10-15 reprises à raison de chaque fois 13 g toutes les 10 minutes à une quantité de 500 g de bonbons.

On a procédé à 4 expériences (35 à 38) et les conditions et résultats portés dans le tableau VIII confirment qu'il est possible de réaliser la dragéification au maltitol de bonbons cuits, à condition de travailler dans les conditions de niveau de saturation établies dans le cas des chewing-gums et dans celui des comprimés.

Tableau VIII

| N° de l'expérience | 35 | 37 | 38 | 39 |
|---|---|---|---|---|
| matière sèche du sirop | 70% | 60% | 55% | 50% |
| température du lit | 30 °C | 40 °C | 30 °C | 30 °C |
| température du sirop | 50 °C | 50 °C | 50 °C | 50 °C |
| niveau de saturation | 1,25 | 0,69 | 0,8 | 0,53 |
| appréciation | o | xx | xxx | x |

## Revendications

1. Produit de confiserie ou pharmaceutique à revêtement sans sucre de nature essentiellement cristalline, caractérisé par le fait que ledit revêtement est obtenu par dragéification dure et qu'il comporte au moins 90% en poids de maltitol.

2. Procédé de préparation d'un produit selon la revendication 1, caractérisé par le fait que l'on applique un sirop de maltitol
— d'une richesse en maltitol supérieure à 92%, de préférence à 95% en poids sur matière sèche, et plus préférentiellement supérieure à 96%,
— d'une teneur en matières sèches de 50 à 70% en poids, de préférence de 55 à 65% en poids et
— d'une température inférieure à 70°C et, de préférence de 45 à 65°,
sur un lit en mouvement de noyaux à dragéifier, la température régnant dans ledit lit de noyaux en mouvement étant inférieure à 55°C et de préférence de 20 à 40°C, l'ensemble des susdites conditions étant choisies, à l'intérieur des gammes susmentionnées, de telle manière que, lorsque le sirop de maltitol arrive au contact des noyaux à dragéifier, c'est-à-dire à la température régnant au sein du lit de noyaux en mouvement, il se trouve à un niveau de saturation de 0,70 à 0,90 et, de préférence, de 0,75 à 0,90.

## Patentansprüche

1. Zuckerwarenprodukt oder pharmazeutisches Erzeugnis mit einer im wesentlichen kristallinen zuckerfreien Beschichtung, dadurch gekennzeichnet, daß die Beschichtung durch Hartdragieren erhalten ist, und zumindest 90 Gew.% Maltit enthält.

2. Verfahren zur Herstellung eines Erzeugnisses entsprechend Anspruch 1, dadurch gekennzeichnet, daß man einen Maltitsirup
— mit einem Gehalt an Maltit über 92 Gew.%, vorzugsweise über 95 Gew.% mit Bezug auf die Trokkensubstanz und in einer noch mehr bevorzugten Weise über 96 Gew.%,
— mit einem Trockensubstanzgehalt von 50 bis 70 Gew.%, vorzugsweise von 55 bis 65 Gew.% und
— mit einer Temperatur unter 70°C und vorzugsweise von 45 bis 65°C,
auf ein bewegtes, aus zu beschichtenden Kernen bestehendes Bett aufbringt, wobei die Temperatur, die in dem bewegten Bett herrscht, unter 55°C liegt, und vorzugsweise von 20 bis 40°C ist, wobei die Gesamtheit der obigen Bedingungen derartig innerhalb der angegebenen Bereiche gewählt wird, daß zu dem Zeitpunkt, an dem der Maltitsirup in Kontakt mit den zu beschichteten Kernen gerät, d. h. die innerhalb des bewegten Bettes aus Kernen herrschende Temperatur erreicht, sich bei einem Sättigungsniveau von 0,70 bis 0,90 und vorzugsweise von 0,75 bis 0,90 befindet.

## Claims

1. Confectionery or pharmaceutical product provided with a sugarless coating of essentially crystalline nature, characterized by the fact that said coating is obtained by hard coating and comprises at least 90% by weight of maltitol.

2. Process for preparing a product according to claim 1, characterized by the fact that a maltitol syrup
— of a richness in maltitol higher than 92%, preferably than 95% by weight to dry matter, and more preferably higher than 96%,
— of dry matter content from 50 to 70% by weight, preferably from 55 to 65% by weight and
— of temperature less than 70°C and, preferably from 45 to 65°C,
is applied on a moving bed of cores to be coated, the temperature existing in said moving core bed being less than 55°C and preferably from 20 to 40°C, all of the abovesaid conditions being selected, within the above-mentioned ranges, so that, when the maltitol syrup arrives in contact with the cores to be coated, that is to say at the temperature existing within the moving core bed, it is at a saturation level of 0.70 to 0.90 and, preferably, from 0.75 to 0.90.